# EUROPEAN PATENT SPECIFICATION

(11) **EP 3 679 516 B1**
(45) Date of publication and mention of the grant of the patent: **06.11.2024**
(21) Application number: 18789684.0
(22) Date of filing: 05.09.2018
(51) Int. Cl.: G06F 18/25, G06V 40/10, G06V 40/70

(54) **WEARABLE AUTHENTICATION DEVICE**
AM KÖRPER TRAGBARE AUTHENTIFIZIERUNGSVORRICHTUNG
DISPOSITIF D'AUTHENTIFICATION VESTIMENTAIRE

(30) Priority: 05.09.2017 GB 201714223
(43) Date of publication of application: 15.07.2020
(73) Proprietor: B-Secur Limited, Belfast, North Ireland BT1 3FE (GB)
(72) Inventor: CONDON, Adrian, Belfast BT4 2GU (GB); JARDINE, David Thomas, Belfast (GB)
(74) Representative: Murgitroyd & Company
(86) International application number: PCT/GB2018/052517
(87) International publication number: WO 2019/048855

(56) References cited:
- EP-A1- 1 610 677
- US-A1- 2017 032 168
- US-A1- 2017 039 358
- "Biometrics in a Data Driven World : Trends, Technologies, and Challenges", 15 December 2016, CRC PRESS LLC, article MITRA SINJINI ET AL: "Biometrics in a Data Driven World : Trends, Technologies, and Challenges", pages: 205 - 206, XP055845744
- SCHUMM JOHANNES ET AL: "Unobtrusive physiological monitoring in an airplane seat", PERSONAL AND UBIQUITOUS COMPUTING, vol. 14, no. 6, 20 February 2010 (2010-02-20), GB, pages 541 - 550, XP093022821, ISSN: 1617-4909, Retrieved from the Internet <URL:http://link.springer.com/article/10.1007/s00779-009-0272-1/fulltext.html> DOI: 10.1007/s00779-009-0272-1

## Description

### Field of disclosure

This invention relates to an authentication system and a method of operating an authentication system.

### Background

As more and more electronic devices are used around the world, there is an increasing need for improved security. User authentication is a process that verifies that a person using a device has rights (is authorized) to utilise the device's resources. A standard approach to user authorization is password verification (and its derivatives, including two-factor authorization, one-use tokens, etc.). A good password should be long enough to provide security from brute-force attacks and unique so that if it is compromised (i.e. known to third parties) it cannot be used in other systems.

One problem with such an arrangement is that a user must remember a great number of long complex passwords. Other ways of user authorization are required. Promising approaches include biometrics-based authentication, which involves novel ways of verifying a user's identity using his or her natural characteristics. Most wide-spread methods include using information from fingerprints, iris or voice, as these are regarded unique to each user. Bioimpedance is also proposed for biometrics-based authentication, as described in WO2001/20538, whereby different impedance measurements are made between different points on a user's hand or body at different frequencies. Electrical signals from the heart can be used to attain data points that are unique to the user.

There are many ways to analyse one's heartbeat, but the most practical approach is analysing the patterns gathered by Electrocardiograph, which records a heart's electric potential changes in time. A longer recording of heartbeat activity is called an electrocardiogram or ECG and is recorded using one or more pairs of electrodes. Each pair measures the change of electrical potential between the points of contact of electrodes. That change is strongly correlated with heart and muscle activity of the subject as the heart beat activity of the human body is stimulated through electrical impulses.

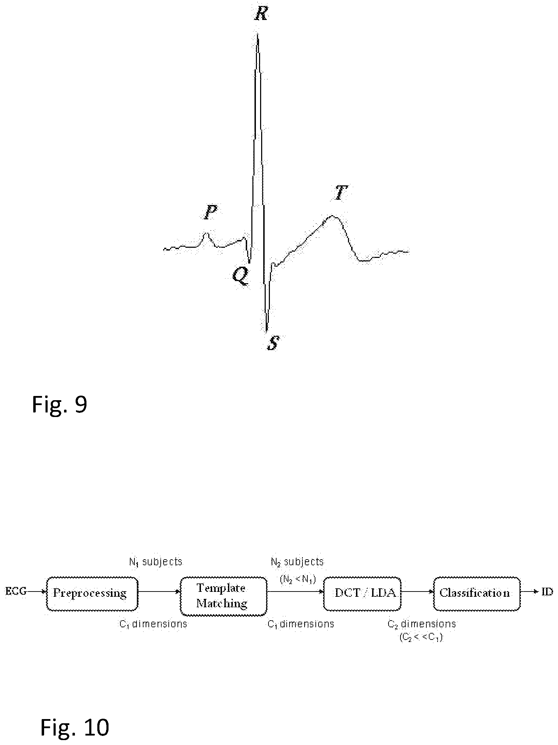

Fig. 9 shows an electrocardiogram signal depicting the electrical potential of the heart over time. The basic elements of a single heart beat are: a P wave generated when the right and left atria of the heart are depolarized; a QRS complex reflecting the depolarization of the right and left ventricles; and a T wave corresponding to the ventricular repolarization. Existing methodologies attempt to characterize an individual by these different elements and their respective sizes, shapes and positions. WO 2008/107684 of Intelesens is an example of such an approach.

For a practical process, the system should be easy to use and be robust. Such an arrangement is described in GB1611963.8 of B-Secur Limited. As an improvement to the traditional challenge/response approach of biometric systems, an approach that facilitates continuous authentication and monitoring would give a better user experience.

Authentication devices are often utilized by users in secure environments. In these scenarios it is important to know who the person is and that they have the capacity to deal with the situation they are in. This applies particularly to users in high stress environments, such as fire-fighter or police.

US 2015/0028996 of Bionym Inc. describes a biometric authentication device in which a wristband is worn on one hand and the user has to touch a finger of the opposite hand to the wristband to collect biometric data. The wristband is then maintained in an authenticated state until the wristband is removed. At this point the authenticated state is lost. US 2015/0028996 speculates on the possibilities of incorporating the biometric device into clothing but it does not present a practical way to perform authentication measurements without action by the user and in a way that receives a valid signal. As discussed above, this may not be viable in scenarios where the user is not able to stop use both hands to authenticate whenever authentication is needed. There are also security concerns regarding devices being transferred to another person without opening the wristband and therefore maintaining the authenticated state for someone who is not entitled to it. There is therefore a need for an improved unobtrusive and passive (on the part of the user) authentication device which also enables measurement or determination of the physiological state of the user.

US 2017/0042485 discloses a method and apparatus for processing a biometric measurement signal using a computing device.

US 2007/0177770 discloses a system and method for identity confirmation using physiologic biometrics to determine a physiologic (i.e. biometric) fingerprint.

US 2017/0179933 discloses a wearable device configured to detect authentication information regarding a user wearing the device.

WO 2016/209376 discloses a method and apparatus for providing a user's physiological context measurements.

US 2016/0335632 discloses a wearable device made with silicone rubber and electronic components. Whereas it discloses combining EDA and heart rate variability measurements to indicate stress, it does not mention how this calculation is done nor does it suggest using them to measure fatigue. It discloses a feedback system, but does not specifically mention using a prompt for detachment and repositioning of sensors.

US 2015/004797 discloses a wearable device and authentication method for performing user authentication using bio-signals.

US 2017/0042485 discloses a method and electronic device for detecting biometric information. However, it does not disclose analysis of the biometric measurements.

US2017/032168 A1 discloses a smart watch capable of acquiring both of fingerprint information and biometric information by a single motion, and a method of operating the same. US 2017/039358 A1 (YUEN SHELTEN GEE JAO [US] ET AL) 9 February 2017 (2017-02-09) discloses a wearable authentication system.

### Summary of Invention

According to the invention there is provided an authentication system and a method of operating an authentication system according to the appended claims.

An authentication system is provided according to claim 1.

A method for the operation of the authentication system is provided according to claim 8.

Thus, for example, it can be verified that the physiological state measured is that of the correct person and is recorded to the correct user profile. For example, if User A puts on a wearable biometric sensing device, the device identifies the user as User A through user authentication (comparing the ECG with a template) and then the user's profile is selected based on this authentication. This then enables the physiological state to be determined using the user profile and recorded to the correct profile. The right thresholds and/or profiles are therefore used for the right person. This has the advantage of ensuring that the device uses the correct thresholds and/or profile when taking measurements and making assessments. For example, if User B put on the same wearable biometric sensing device, the device would identify the user as User B, rather than User A or any other user. This is done through user authentication and then User B's profile is selected based on this authentication. The wearable biometric sensing device is able to store the profiles for multiple users and the correct profile is brought up when the user is authenticated.

Various embodiments and aspects of the present invention are described without limitation below, with reference to the accompanying figures.

### Brief description of the drawings

*Figure 1* depicts a line drawing of an embodiment of the invention, showing a top, sensors and a wearable computing means.
*Figure 2* depicts a line drawing of an embodiment of the showing a bra, sensors and a wearable computing means.
*Figure 3* depicts a line drawing of an embodiment of the invention, showing underwear, sensors and a wearable computing means.
*Figures 4a* and 4*b* depict line drawing of an embodiment of the invention, showing a material strip, sensors and attachment means.
*Figure 4c* depicts a line drawing of the invention, showing a car seat, computing means and sensors.
*Figure 5* depicts a block diagram of an embodiment of the invention, showing a user device and a remote terminal.
*Figure 6a* depicts a block diagram of an embodiment of the invention, showing an authentication system, wearable apparatus and a remote terminal.
*Figure 6b* depicts a block diagram of an embodiment of the invention, showing an authentication system, wearable apparatus, wearable computing means and a remote terminal.
*Figure 7* depicts a method flow diagram of an embodiment of the invention.
*Figure 8* depicts a data flow diagram of an embodiment of the invention.
*Figure 9* shows an electrocardiogram signal depicting the electrical potential of a heart beat over time.
*Figure 10* depicts an autocorrelation based feature extraction approach as described in ECG Based Recognition Using Second Order Statistics by F. Agrafioti Detailed description of a preferred embodiment

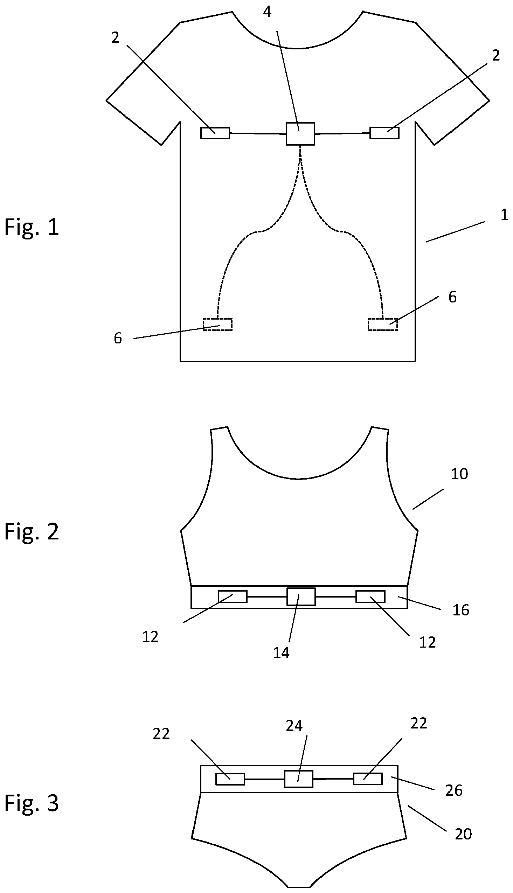

Figure 1 shows an elasticated (e.g. "compression-type") top 1having: sensors 2; a wearable computing means 4 and optional sensors 6. In a preferred embodiment, the sensors 2 are biometric sensors 2. In the preferred embodiment, the types of the biometric sensors 2 are electrocardiogram (ECG) electrodes and Electrodermal Activity (EDA) electrodes, where the ECG electrodes and the EDA electrodes are the same electrodes. I.e. some of the electrodes are connected to an amplifier for ECG measurement and others are connected to an amplifier for EDA measurement (which may be a different amplifier) while some of the electrodes are common to both amplifiers. When electrodes are common to both amplifiers, it enables the device to use the same sensor for multiple purposes, The electrodes are capable of measuring the biometric data of a wearer (or user), for example ECG and EDA data, and transmitting the data to the wearable computing means 4 via a wired connection. In an alternative embodiment, the sensing element communicates wirelessly to the computing module in the same wearable garment or a separate wearable computing module.

In some embodiments the optional sensors 6 are sensors of the same type as the sensors 2 for more measurement points to enable more accurate readings. In other embodiments the optional sensors 6 are sensors of a different type to the sensors 2. The different types of sensors 6 include, for example, sensors to measure respiration rates through use of Doppler ultrasound to detect the movement of internal organs, photoplethysmogram (PPG) sensors and motion sensors. A location sensor and/or an altitude sensor may be provided. The location sensor may be a satellite positioning receiver such as a GPS receiver (or its equivalent). Other RF receivers can be used for location determination, including Bluetooth low energy (BLE), and various wireless (including UHF) receive signal strength indicators.

In the preferred embodiment, processing is carried out by the wearable computing means 4 includes an authentication process (for example, based on the data from the ECG and EDA electrodes) and a physiological state determination process (for example, based on data from the ECG and EDA electrodes). The physiological state of a user is preferably a state as determined by a classifier, using physiological information as an input. Examples include user stress level, the user duress level, and/or the user fatigue level. The physiological state of a user may be a result of the combination of all the physiological information to give an overall user physiological state.

Combining different types of sensor 6 can provide a remote terminal operator with more information regarding a user of the top 1. Furthermore, combining different types of sensor 6 allows for improved accuracy in certain biometric readings. The combination enables artefacts in reading results to be identified and ignored. In a preferred embodiment the use of ECG authentication with EDA stress level monitoring enables a user of the top 1 to be identified and authenticated, and can also indicate their psychological state. The ECG signal can also be used to measure BPM which can be combined with EDA measurements for a more accurate measure of stress levels.

Alternatively, EDA measurements can be combined with heart rate variability (HRV) measurements to give a measure of fatigue. For example, time domain HRV analysis can be conducted on beat-to-beat or normal-to-normal (NN) intervals between successive normal ECG complexes. The standard deviation of the NN interval and root mean square of successive differences can be derived, as can the ratio of the total number of NN intervals (pNN50), where NN50 is the number of pairs of successive NNs that differ by more than 50ms. These various measurement methods can be used to estimate high-frequency (HF) and low-frequency (LF) variations in the heart rate. The ratio of LF/HF variations is a particularly useful indicator of fatigue (where a value below 3 is normal, a value of 3.5 indicates fatigue and a value above 4.0 indicates drowsiness).

The combination of ECG and EDA data along with motion sensor data enables certain determinations to be made or assumptions to be removed regarding the user baseline levels for ECG (including HRV) and EDA data.

Figure 2 shows a bra 100 having: an elastic band 16; sensors 12; and a wearable computing means 14. In a preferred embodiment the sensors 12 are embedded in the elastic band 16. In an alternative embodiment the elastic band 16 is conductive elastic and is combined with the sensors 12.

In the preferred embodiment, the sensors 12 are biometric sensors 12. Types of biometric sensors 12 are ECG electrodes and EDA electrodes. In the preferred embodiment, the biometric sensors 12 are capable of measuring the biometric data of a wearer (or user), for example ECG and EDA data, and transmitting the data to the wearable computing means 14 via a wired connection. In an alternative embodiment, the data is transmitted to the wearable computing means 14 via a wireless connection.

In the preferred embodiment, processing carried out by the wearable computing means 14 includes an authentication process (for example, based on the data from the ECG and EDA electrodes) and a physiological state (or "wellness") determination process (for example, based on data from the ECG and EDA electrodes). The authentication process is carried out by comparing the measured user ECG with a template and the physiological state determination process is carried out by comparing one or more of the user's measured ECG, EDA or EMG with a threshold or profile.

Figure 3 shows underwear 20 having: an elastic band 26; sensors 22; and a wearable computing means 24. In a preferred embodiment the sensors 22 are embedded in the elastic band 26. In an alternative embodiment the elastic band 26 is conductive elastic and is combined with the sensors 22.

In the preferred embodiment, the sensors 22 are biometric sensors 22. Types of biometric sensors 22 are ECG electrodes and EDA electrodes. In the preferred embodiment, the biometric sensors 22 are capable of measuring the biometric data of a wearer (or user), for example ECG and EDA data, and transmitting the data to the wearable computing means 24 via a wired connection. In an alternative embodiment, the data is transmitted to the wearable computing means 24 via a wireless connection.

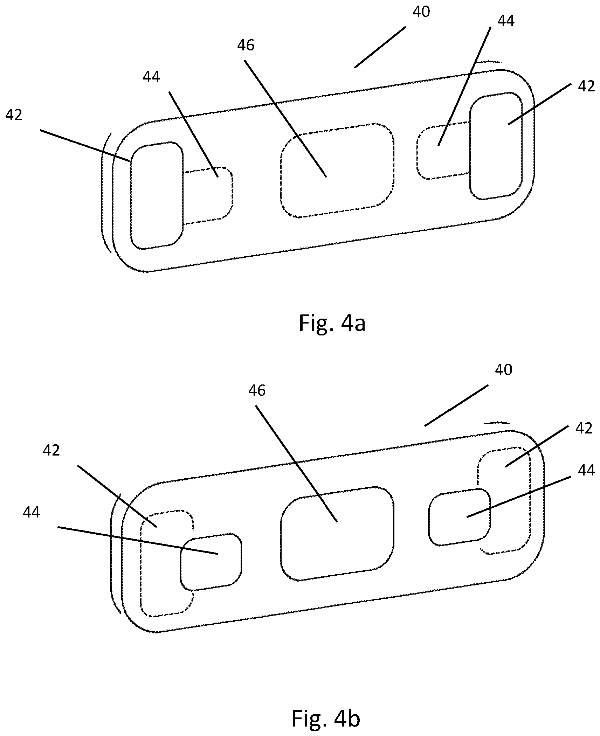

Figures 4a and 4b shows a material (preferably elasticated material) strip 40 having: attachment means 42; sensors 44; and wearable computing means 46. Figure 4b shows the other side of the material strip 40 in Figure 4a.

In a preferred embodiment, the attachment means 42 enables the material strip 40 to be temporarily secured where needed. The attachment means 42 can, for example, be clips, barb connections, buttons, press fasteners, hook and eye fasteners, fabric hook and loop fasteners or temporary adhesive. In some embodiments, the material strip 40 is temporarily secured by the attachment means 42 to an elastic component of a piece of clothing.

In some embodiments the wearable computing means 46 has a transducer or feedback element which enables the user to be alerted when the sensors 44 have become detached and need repositioning.

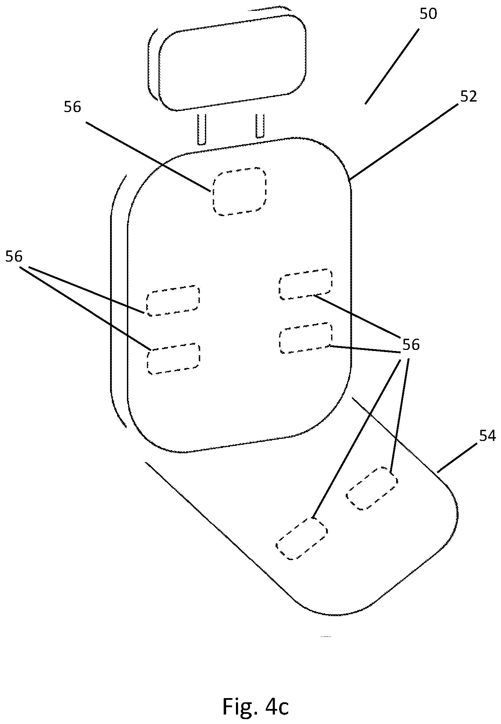

Figure 4c shows a car seat 50 having: a car seat back 52, a car seat base 54, computing means 56, and sensors 56.

In a preferred embodiment, the sensors 56 are biometric sensors. In the preferred embodiment, the types of the biometric sensors 56 are ECG electrodes and EDA electrodes, where the ECG electrodes and EDA electrodes are the same electrodes. The electrodes are capable of measuring the biometric data of a user, for example ECG and EDA data, and transmitting the data to the computing means 56 via a wired connection. In an alternative embodiment, the sensing element communicates wirelessly to the computing module 56 in the same car seat 50 or a separate wearable computing module.

In some embodiments only the car seat back 52 has sensors 56. In other embodiments only the car seat base 54 has sensors 56 and in other embodiments both the car seat back 52 and car seat base 54 have sensors 56. The sensors 56 are optimally positioned within the car seat back 52 and car seat base 54 so that they are as close to the skin of the user as possible.

In some embodiments all of the sensors 56 are sensors of the same type for more measurement points to enable more accurate readings. In other embodiments some of the sensors 56 are sensors of a different type.

In a preferred embodiment, the sensors 56 take non-contact measurements and do not need to be in contact with the user's skin, merely as close to it as possible. In a preferred embodiment, the non-contact measurements taken by the sensors 56 are capacitive measurements. It is possible to take a measurement of ECG without direct skin contact. The sensors 56 are positioned very close to the skin and the electrical signal can capacitively couple to the sensor 56.

In the preferred embodiment, processing is carried out by the computing means 56 includes an authentication process (for example, based on the data from the ECG and EDA electrodes) and a physiological state determination process (for example, based on data from the ECG and EDA electrodes).

In some embodiments the computing means 56 has a transducer or feedback element which enables the user to be alerted when the body needs repositioning with respect to the sensors 56.

In the preferred embodiment, when seated in the car seat, the user is reasonably stationary. By using time-based filters it is possible to filter the ECG signal from other longer term changes arising from the user, for example, shifting position in the seat.

### Biometric Sensors

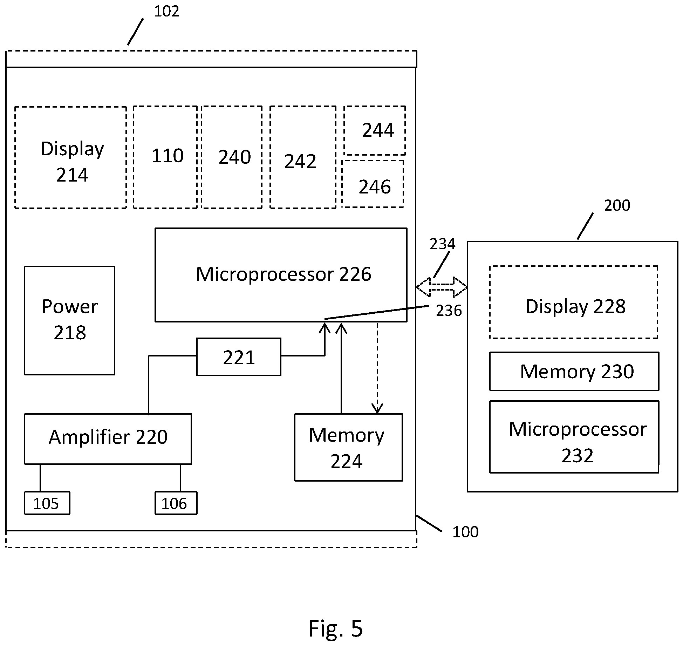

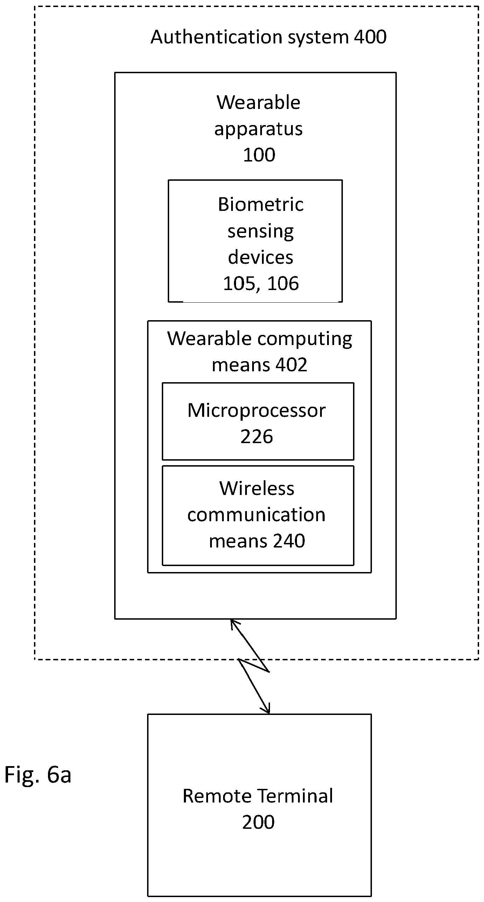

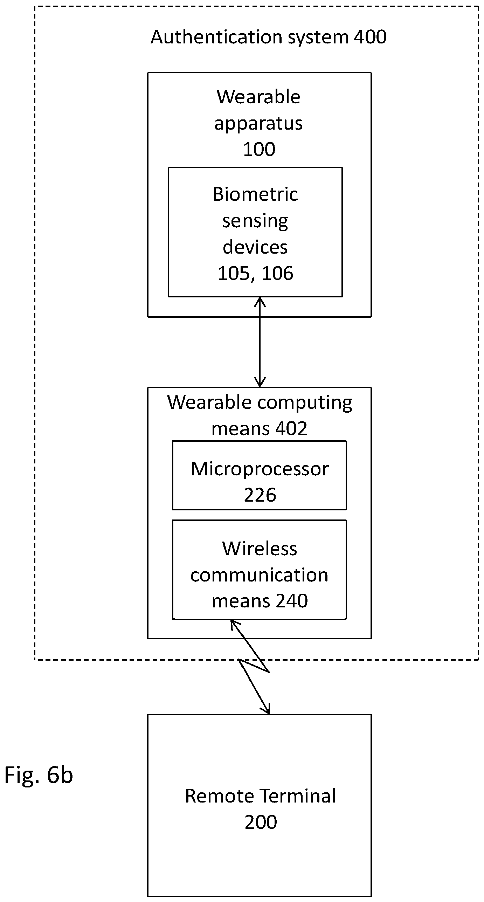

Figure 5 shows an illustrative block diagram representing the hardware for capturing and processing a user's ECG sample authentication. Figure 6 shows a wearable apparatus 100 having: sensors 105, 106; a power supply 218; an amplifier 220; hardware filters 221; a memory module 224; a microprocessor 226; a wireless communication means/transmitter 240 and optional display 214, motion sensor 110, attachment means 102, locator module 242, altitude sensor 244 and temperature sensor 246. Figure 6 also shows a remote terminal 200 including a memory 230, a microprocessor 232 and an optional display 228. The wearable apparatus 100 and the remote terminal 200 optionally communicate 234 with each other. The remote terminal 200 may alternatively be a server or an operator terminal. If the remote terminal 200 is an operator terminal, the operator terminal may optionally communicate with a different server.

In a preferred embodiment, the wearable apparatus 100 has a power source from the power supply 218. In an alternative embodiment, the wearable apparatus 100 receives power from other means, for example from the wearable computing means or from motion.

In the preferred embodiment, the sensors 105, 106 are biometric sensors 105, 106. In the preferred embodiment that types of biometric sensors 105, 106 are electrocardiogram (ECG) electrodes and Electrodermal Activity (EDA) electrodes. In the preferred embodiment, the biometric sensors 105, 106 are capable of measuring the biometric data of a wearer (or user), for example ECG and EDA data, and transmitting the data to the wearable computing means 402 via a wired connection, as shown in Figure 6a. In an alternative embodiment, the data is transmitted to the wearable computing means 402 via a wireless connection, as shown in Figure 6b. The microprocessor 226 processes the data received from the sensors 105, 106, and transmits the processed data to the remote terminal 200 via the wireless communication means 240.

As an alternative to a wireless connection, the user can be required to plug in a tether or dock with a docking station to download readings, e.g. for use by a clinician to perform direct diagnostics. A tether or docking feature can overcome limitations in terms of memory or data transfer rates and serves as a backup if the wireless connection fails.

In the preferred embodiment, the processing carried out by the microprocessor 226 includes an authentication process (for example, based on the ECG data from the sensors 105, 106) and a physiological state determination process (for example, based on EDA data from the electrodes 105, 106). In this preferred embodiment the remote terminal 200 may alternatively be a server 200. When the processing has been carried out by the microprocessor 226 the resulting data may be transmitted to the server 200. In an alternative embodiment, the processing carried out by the microprocessor 226 is limited, for example processing the data for efficient transmission to the remote terminal 200 (e.g. putting the data into a readable file format, and allocating headers where appropriate). In this alternative embodiment, an authentication process and physiological state determination process based on the data is carried out at the remote terminal 200.

Note that the sensing electronics (amplifiers, filters etc.) may be integrated with the electrodes, for example using a flexible printed circuit board (PCB). Other developments may include electronics components integrated into actual conductive fibres, thereby avoiding any need for PCBs etc.

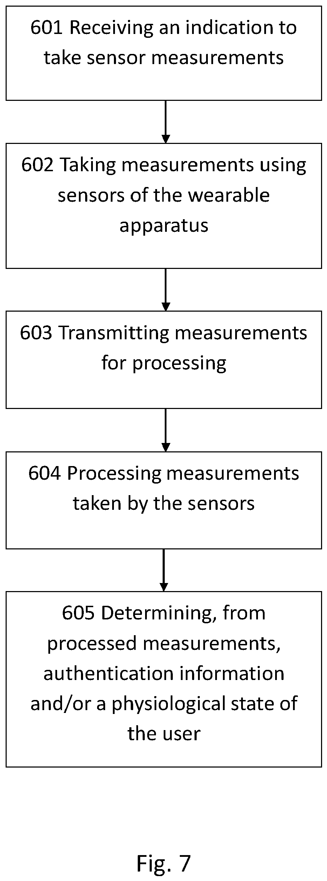

Figure 7 shows a method of operating an authentication system for determining authentication information and physiological information in accordance with a preferred embodiment. An authentication system is a system for authenticating a user of a wearable apparatus. In a preferred embodiment the authentication system also includes determining the physiological state of the user.

At 601 an indication to take sensor measurements is received by the biometric sensing devices 105, 106 from the microprocessor 226. The microprocessor 226 may transmit the indication to the biometric sensing devices 105, 106 based on one or more of a number of triggers. For example, a trigger may be at a regular interval (for example, every 5 seconds) or when contact between one or more sensors is restored with a previously authenticated user following loss of an authenticated state (e.g. in a situation where a sensor on the wearable device detaches from the user temporarily as the user moves, the authenticated state will be lost upon detachment, and subsequently contact with the sensor will be restored). In some embodiments, the trigger may be an off/on switch on the wearable apparatus 100 or significant movement detected by the motion sensor 110. In some embodiments, the indication may be received by the biometric sensing devices 105, 106 from the remote terminal 200, via the wireless communication means 240 and wearable computing means 402. The remote terminal 200 may transmit the indication on instruction from an operator when the remote terminal is an operator terminal. In some embodiments, the indication may be received by the biometric sensing devices 105, 106 from a user interaction with the wearable computing means, for example, pushing a button or activating an authentication procedure.

Once the indication has been received from the microprocessor 226 or remote terminal 200, the sensors 105, 106 take measurements at 602. In a preferred embodiment, measurements may be taken using biometric sensors, for example, ECG electrodes, EDA electrodes, Doppler ultrasound devices photoplethysmogram (PPG) sensors and/or thermal sensors for skin temperature. Measurements are also taken using motion sensors in addition to the biometric sensors 105, 106

Once measurements have been taken, they can be transmitted to a relevant processor for processing (603). In a preferred embodiment, the measurements taken by the sensors are transmitted to the wearable computing means 402, to which they have a wired connection. At 604, the measurements transmitted by the sensors to the wearable computing means 402 are processed by the microprocessor 226 to determine instantaneous biometric information of the user. For example, in relation to ECG measurements, the microprocessor 226 determines an instantaneous user ECG and in relation to the EDA measurements, the microprocessor 226 determines an impedance or conductance measurement of the user's skin.

The microprocessor 226 uses the instantaneous biometric information of the user to authenticate the user and determine the physiological state of the user at 605. The microprocessor 226 performs an authentication process based on the ECG data from the electrodes 105, 106 and a physiological state determination process based on EDA data from the electrodes 105, 106. ECG data can also be used to assist in the physiological state determination process and EDA data can also be used to assist in the authentication process. In relation to the authentication process, a user will have a unique ECG signal that can be compared to a stored ECG signal to ensure the user of the wearable apparatus 100 is who they purport to be. A physiological state determination process based on the EDA determines user stress levels and includes comparing an impedance or conductance measurement from the electrodes with a predefined level, preferably calibrated for the user.

A number of algorithms may be used including ECG detection, BPM calculation, ECG authentication, Heart Rate Variability measurements. Examples of suitable algorithms can be found in GB1611963.8. When the processing has been carried out by the microprocessor 226 the resulting data may be transmitted to the remote terminal 200.

An indication of the result of user authentication (authentication information) and the determined physiological state of the user is transmitted to the remote terminal 200. The remote terminal 200 may be an operator terminal or a server. Alternatively, the indication of the result of user authentication and the determined physiological state of the user is displayed on the display 214 of the wearable apparatus.

In some alternative embodiments, the measurements taken by the sensors 105, 106 may be transmitted to the remote terminal 200 for processing via the wearable computing means 402. The measurements are passed to the wearable computing means 402, whereupon they are transmitted to the remote terminal 200 by the wireless communication means 240 at 603. In some such embodiments, the measurements taken by the sensors 105, 106 are processed by the microprocessor 226 of the wearable communication means 402 to format data for efficient transmission to the remote terminal 200 (e.g. putting the data into a readable file format, allocating headers where appropriate and including security implementation for (for example) key exchange with remoter terminal 200) at 604. The processed measurements are transmitted to the remote terminal. The authentication of the user and determination of the physiological state of the user at 605 is then carried out at the remote terminal 200 in the alternative embodiment.

When the remote terminal 200 is an operator terminal, the authentication information can be, for example, monitored to ensure that the user has the correct security clearance to access different areas of a building, such as secure areas of an airport or bank. The determined physiological state may, for example, be utilised alongside the authentication information to ascertain if, even though someone has authenticated, they are under duress. In the example of an authenticated user having correct security clearance, it is conceivable that they are made to access a secure location, such as a bank vault, under duress. Therefore, the determined physiological state of the user and the authentication information may, for example, be utilised alongside each other to determine if the user should be authenticated when in a determined physiological state. For example, not authenticating someone to enter a secure location, even if they are the correct user, but they are under duress. If the remote terminal 200 has both authentication information and the determined physiological state, it can take appropriate action (such as informing the relevant authorities) in such situations, for example acting as an automatic trigger input for security alarms. In another example, the determined physiological state of the user may be used to indicate when a user is not fit for the task they are carrying out, such as the monitoring of an X-ray security machine in an airport. In this example the user may not be fit for the task if they are not alert. In another example, the appropriate action is activating a trigger. In this example, the trigger may be activated when the user is authenticated but the determined user physiological state is outside a predetermined range, such as a normal range of stress levels. The trigger may activate an alarm, a camera, or initiate a communication to a remote service such as a relevant authority.

In another example, a user has authenticated to gain access to their bank account at an ATM but their biometric information indicates that they are stressed and likely under duress. In this situation the ATM may activate a camera to record the situation or call the police. In yet another example, a fire-fighter may be in a dangerous situation. The authentication information enables a terminal operator to ascertain the identity of the fire-fighter, his or her physiological/psychological state and his/her degree of fatigue, thereby indicating whether he or she should be removed from the situation or assisted.

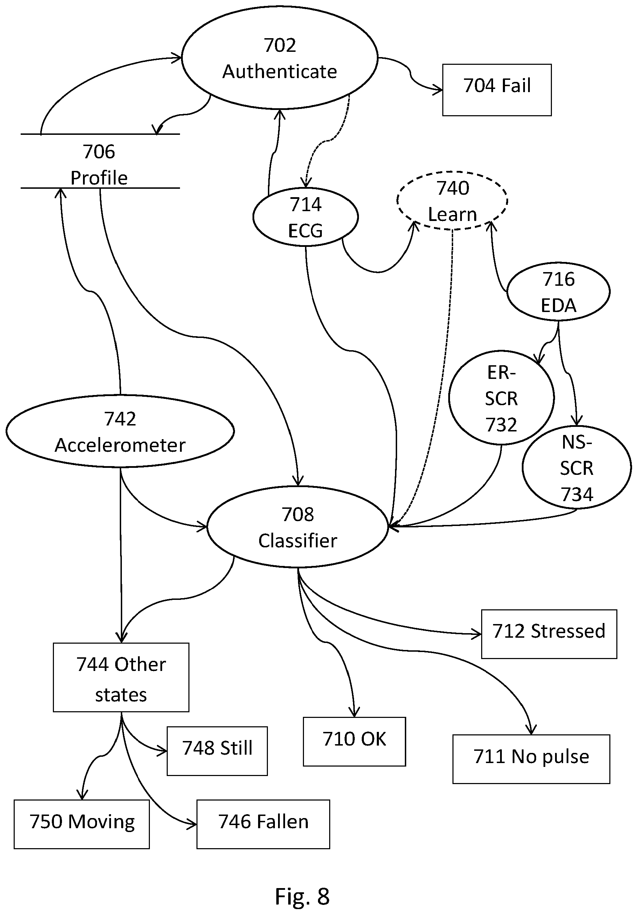

Figure 8 shows a data flow diagram of an embodiment of the invention. ECG measurements are taken at 714 and Electrodermal Activity (EDA) 716 measurements are taken at 716. In a preferred embodiment EDA measurements 716 are analysed to gather Event-Related Skin Conductance Response (ER-SCR) 732 and Non-Stimulus-locked Skin Conductance Response (NS-SCR) 734 data. The ECG data authenticated at 702 based on a stored or pre-enrolled user profile 706. The user profile 706 may be updated to take into account slight variations in the authenticated user's profile. If the ECG data is not authenticated 702 against the user profile 706, the authentication fails 704. If the user is authenticated 702, the EGC data and EDA data is communicated to a classifier or some other method of comparing data.

The classifier performs analysis 708 of the data to ascertain the state of the authenticated user. In a preferred embodiment the classifier receives the user profile 706 to use as a baseline in the classification analysis.

In a preferred embodiment the potential states of the user are "high stress" 712, "low or medium stress" 710 (or other gradation of physiological state depending on the use case) and (optionally) "no pulse" 711. In some embodiments additional data may be used to ascertain other user states 744. Other states 744 include the user being "still" 748, "the user has fallen" 746 or "the user is moving" 750. In some embodiments the additional data needed for these states is received from an accelerometer 742 which measures the movements of the user.

In an example scenario, an elderly person is horizontal and is not moving but he or she has a high level of stress. In this scenario, the appropriate authority can be alerted to assist the elderly person. The RF capability of the clothing or device can also help determine a situation - e.g. GPS plus accelerometer. For example the person is on his or her back, and has not moved for a certain period of time, thereby raising an alarm.

In some embodiments a learning process 740 takes place. ECG and EDA (and/or EMG) data is used to learn how the user acts over time. A profile of the user is built up. In some embodiments the learnt profile of the user can include the extent of the user's reaction to different scenarios, if there are patterns in the user being in certain states at certain time and if there are particular times when the activity of the user results in poor ECG and EDA measurements being taken. In a preferred embodiment, this learnt profile is utilized by the classifier 708 to ensure measurements used in the analysis are optimal (e.g. filtering out measurements at times that, from the historical profile, are known to give unreliable measurements) and allow trends in the user state to be highlighted. These trends in the user state can be used to determine when the optimal time is to measure values, when to discard values that are commonly poor or if the user reacts in a certain (positive or negative) way to certain scenarios. For example, if a user is highly fatigued at the end of a shift and they are unsafe to work, this trend can be highlighted in the user's profile and utilized.

In some embodiments, several authentication devices can communicate a single user's learnt profile to a terminal. This enables multiple devices to contribute to the user's learnt profile and hence the profile is built up based on data from many different devices at the same time or at different times. In some embodiments the multiple devices may be sportswear, sleep wear, jewellery and everyday accessories, or work-specific uniform or accessories. This ensures that the user's learnt profile may be accurate and useful in any scenario the user is in. For example, a user wears a sportswear top when running, which records data and contributes to the user's profile stored at a terminal. The user may wear a wristwatch during the day and at night. This also records data and contributes to the user's profile stored at the terminal. This data can be from multiple devices worn simultaneously and hence the data can be taken simultaneously or from multiple devices worn at different times and hence the data is taken at different times. Each device contributes to the user's learnt profile stored at the terminal. Alternatively, each device may develop its own individual user profile and communicate this to the terminal.

The additional sensory elements can also be used in conjunction or stand alone to the ECG and EDA. For example understanding the physical response of the user to a stressful or fatigue event could be useful data. The combination of ECG, GPS, HRV and EDA contribute to this.

The same electrodes in a wearable can be used to determine EMG (electromyogram) noise. This can help in determining a condition or state of a user. Surface EMG can be recorded by measuring a potential difference between a pair of electrodes or from an array of electrodes and by measuring potential differences between them.

Machine learning algorithms can be applied to EMG and to the accelerometer in combination. These elements contribute to gait analysis. Machine learning analysis of all these elements can be used to help confirm the identity of the user.

One set of electrodes can be used for ECG, EDA and EMG. Alternatively the wearable may have a certain set for one element and a separate set for another
In embodiments that measure ECG, electrodes 105, 106 will be positioned on the user's skin to collect information from a user's heartbeat. For example, an ECG electrode can be located in front of the user's heart, at the small of the back or on the user's arms, legs or chest. ECG electrodes 105, 106 detect viable ECG signals when placed in direct contact with skin. The ECG electrodes 105, 106 may be electrodes comprising any conductive material including conductive fabrics, coating and/or metals. The ECG signal collected can be used to authenticate a user. The ECG signal can also be used, for example, to measure the change in heart Beats Per Minute (BPM), comparing a user's resting BPM to the current BPM and /or Heart Rate Variability.

The EDA electrodes are used to detect the changes in skin conductance of the body that indicate variations in the sympathetic and parasympathetic nervous systems. A low constant voltage is applied to two EDA electrodes. The voltage difference between the two electrodes is then measured and from this the associated skin conductance can be calculated. The change in the value is directly related to the physiological changes in the body due to for example, stress or exercise where for example, the moisture levels in the skin change when sweat glands are more active.

As the body becomes more physiologically aroused, the activity of the sweat glands increases, which increases the electrical conductance of the skin. There are two main types of EDA activity. Phasic activity refers to a rapidly fluctuating signal related to stimulated physiological arousal, whereas tonic activity refers to a slowly fluctuating signal that is related to baseline physiological arousal.

A change in EDA activity occurs when an event is presented, this is called an Event-Related Skin Conductance Response (ER-SCR). This activity generally occurs between 1 and 5 seconds after the onset of emotional stimuli. The raw values of EDA or the presence of a EDA peak can be examined. By contrast, Non-Stimulus-locked Skin Conductance Response (NS-SCR) refers to a change in EDA activity that occurs over a longer time period. The frequency of NS-SCRS is typically 1-3 per minute during rest and over 20 per minute in high arousal situations. These responses occur spontaneously in the body and are not related to any eliciting stimulus. The number of EDA peaks, and the distance between then can be examined. The peaks refer to the EDA signal intensity passing a certain threshold. The more peaks there are, the more physiologically aroused the person is.

Change in EDA can be used to measure the stress or fatigue levels of the user. This is useful when the user may be in an environment that requires their full attention, requires them to be calm or may be dangerous. For example, a security guard, a fire fighter, a policeman or someone in a secure workplace. Measuring a user's stress levels is beneficial in that they can be monitored for signs that they are under duress, are not fit for the work they are carrying out or are under significant mental stress.

Artefacts in the EDA measurement can be reduced by signal smoothing algorithms. However, some signal smoothing algorithms require down-sampling. Such down-sampling is always going to be restricted if the original sample rate was itself quite low. With high sample rates one can remove a host of artefacts without altering the signal shape markedly and still have plenty of samples remaining for an appropriate analysis (the ideal scenario).

The signal can be inspected for artefacts such as periods of poor contact or noise interference (an example of which is sharp square-wave spiking) that may reflect contamination from an artificial source. These artefacts can be removed by digital filtering. On rare occasions noise and/or artefacts can be too excessive and so the data should be discarded. An acquisition rate of between 500 and 2000 samples per second is preferable for sampling.

Alternatively, if the measurement is combined with different indicators, such as ECG measurements and BPM measurements, artefacts can be identified and removed. Artefacts may occur due to movement of the user or the wearable apparatus 100.

EDA can also be used to detect when the wearable apparatus 100 has been removed, and hence the authenticated state of the wearable apparatus 100 should be removed. This is done by detecting when the EDA measurement is outside the normal range (e.g. normal for the authenticated user) and hence there is no skin contact with the EDA electrodes. This ensures that the wearable apparatus 100 cannot be authenticated by one user and then transferred to a different user who is not eligible for the authenticated state. The authenticated state can only be reinstated when the wearable apparatus 100 is re-authenticated. In an alternative embodiment, the detection that the wearable apparatus 100 has been removed can also be done by different types of biometric sensors 105, 106 in the same manner.

In some embodiments, there are further types of biometric sensors 105, 106. Different types of biometric sensors 105, 106 may be used to measure respiration rates, including use of Doppler ultrasound to detect the movement of internal organs and/or derived from ECG in additional to utilising impedance measurement of electrodes. The chest movement of the user during respiration affects the impedance response of any electrodes on the chest. Any additional electrodes on the back can be cross referenced to the measurement of the chest electrodes. The chest heaving and relaxing causes the measureable change.

In some embodiments the biometric sensors 105, 106 are not positioned on the user's skin, only very close to it. This can be temporary due to movement of the user or permanent due to fit of the wearable system against the user's skin or by design. For example, in a car seat 50. The car seat 50 can have sensors in both the car seat back 52 and the car seat base 54 in order to enable optimal taking of measurements. It is possible to take a measurement of ECG without direct skin contact. The biometric sensors 105, 106 are positioned very close to the skin and the electrical signal can capacitively couple to the sensor. As the biometric sensors 105, 106 do not have to be touching the skin, this enables quick and easy measurement of the user to authenticate the user when they are, for example, sitting on a car seat.

In some embodiments, the measurements taken by the biometric sensors are used to authenticate the user and/or determine their physiological state. When the user is authenticated this can enable access to the car, such as the ability to start the engine or access information stored by the car. However, if the user's physiological state is not within a threshold or usual profile, even if they have been authenticated, they can be denied access to the car. For example, in situations where the user is under duress or has high levels of stress when trying to drive or use the car. This is ensures the safety of the user and those around the car to ensure that the user is fit to drive.

Photoplethysmogram (PPG) sensors are another type of biometric sensor that may be used. PPG uses electrical signals derived from light reflected due to changes in blood flow during heart activity. PPG has also been widely used to estimate respiratory rate due to its simplicity and non-invasive measurement capability. The PPG signal contains components that are synchronous with respiratory and cardiac rhythms. Respiration is known to modulate the frequency and amplitude of PPG signals. The occurrence of temporal variations in frequency and amplitude is characteristic of the respiration produced by most animals, including humans. Thus, respiratory rates in the normal breathing range can be accurately obtained by computing spectral analysis of the amplitude modulation (AM) and frequency modulation (FM) time series.

Combining different types of biometric sensor 105, 106, can provide a remote terminal operator with more information regarding a user of the wearable apparatus 100. Furthermore, combining different types of biometric sensor 105, 106 allows for improved accuracy in certain biometric readings. The combination enables artefacts in reading results to be identified and ignored. For example, the use of ECG authentication with EDA stress level monitoring enables a user of the wearable apparatus 100 to be identified and authenticated, and can also indicate their physiological state. The ECG signal can also be used to measure BPM which can be combined with EDA stress level measurements for a more accurate measure of stress levels.

### Additional Wearable Apparatus Modules

An optional locator module 242 can be used to detect the location of the user. In a preferred embodiment, the locator module 242 is a GPS module. The GPS module communicates with one or more satellites, calculates the location of the wearable apparatus 100 and then preferably communicates the location to the remote terminal 200 via the wireless communication means 240. This ensures the user can be located, enabling others to locate and assist them or alternatively know if they are not where they are meant to be. With regard to emergency services, for example, if the user is in a particularly stressful environment, knowing the location of the user at the remote terminal 200 can allow assistance to be dispatched to the correct location more efficiently. In an alternative embodiment, the locator module is a Bluetooth Low Energy (BLE) beacon.

Other means for determining the location of a user are within the scope of the present invention. A network device may determine a location of a user and communicate it to the remote terminal. The determination may be done by determining what wireless access points the network device is within range of, triangulation between wireless access points to provide an approximation of the location of the user, or through facial recognition in a network of cameras. For example, if a user is taking money from an Automated Telling Machine (ATM), there is often a camera that can be used to indicate that the user is present at the ATM. The location of the camera therefore indicates an approximate location of the user. A motion sensor 110 is used to detect motion of the body, which can be utilised, for example, to calculate body positioning or gait analysis or to prompt the optional GPS module 242 to communicate an updated location to the remote terminal 200. The motion sensor 110 can be an accelerometer, gyroscope, magnetometer and/or an overall nine-axis inertial motion sensor. Such information are used in combination with readings from the biometric sensors to more accurately determine the user's physiological state. For example, if it is known that the user is walking quickly, they can be expected to have a higher heart rate and can be expected to sweat, thereby increasing conductivity across the skin. Machine learning algorithms across the sensors 105, 106 can be used to determine or remove certain assumptions. For example, higher BPM is acceptable, algorithm can deploy certain techniques to remove motion artefact noise and/or impedance measurement at sensor 105, 106 can be adjusted. In an example scenario alarms regarding high levels of user stress may be suppressed because the user is walking quickly or running. In another example scenario the sensors 105, 106 may not take measurements at a particular time due to high levels of motion and the potential of poor resulting measurements.

An optional altitude sensor 244 can be used to monitoring the altitude of the user. The altitude sensor 244 may be a barometer. The sensor 244 may be utilized, for example, to monitor which floor of a building the user is on. The data from this altitude sensor 244 can also be used to determine or remove certain assumptions when determining the user's physiological state. For example, when it is detected that a user's altitude is increasing and they have a high heart rate, it can be assumed that they are exerting themselves by going up a staircase. This can be taken into account when determining the user's physiological state. If it is known that the user is walking up a staircase, they can be expected to have a higher heart rate and can be expected to sweat, thereby increasing conductivity across the skin.

Machine learning algorithms across the sensors can be used to determine or remove certain assumptions. Examples of such assumptions or determinations are:
(i) determining that a higher BPM is acceptable;
(ii) determining to remove motion artefact noise by the algorithm deploying certain techniques; and
(iii) adjusting the impedance measurement at the sensor.

In an example scenario, alarms regarding high levels of user stress may be suppressed because the user is walking up a staircase. In another example scenario, the sensors may not take measurements at a particular time due to high levels of motion and the potential of poor resulting measurements.

An optional temperature sensor 246 can be used to monitor the skin temperature of the user and/or the air temperature. The data from this temperature sensor 246 can be used to determine or remove certain assumptions when determining the user's physiological state. For example, when a user has a higher skin temperature than normal or the air temperature is higher than usual it can be expected that the conductivity would go up because the user is exercising or is in a hot location where as if the person was cool but conductivity is increasing then it can be assumed that they are more likely to be stressed.

In an example scenario, the user's skin temperature suddenly drops and remains suppressed. This, combined with a physiological change, such as a change in heart rate and/or breathing, can be indicative of falling into cold water.

### Wearable Computing Means

As shown in Figures 7 and 8, the wireless communication means 240 can communicate with the remote terminal 200 by any suitable wireless communication link. For example, the wireless communication link may be, Bluetooth, WiFi, NFC or Radio Frequency.

The filters 221 preferably comprise, in sequence, a high pass filter set at a first threshold cut-off and a low pass filter set at a second threshold cut-off, plus (optionally) a notch filter set at a third threshold for filtering any noise from mains supply. In a preferred embodiment, the first threshold may be 0.5Hz but more preferably 0.05Hz, the second threshold may be 150Hz and the third threshold may be 50Hz. The amplifier 220 preferably amplifies the signal measured by the biometric sensors 105, 106. In a preferred embodiment, the amplification carried out by the amplifier 220 and the filtering carried out by the filter 221 together pre-process the signal measured by the biometric sensors 105, 106. In an alternative embodiment, adaptive filtering and application (gain) can utilised.

Depending on the resolution of the analog-to-digital convertor of the microprocessor, a number of hardware filtering components can be reduced, and the major part of the signal processing occurs in software, e.g. through notch filters etc. This also removes the need to have specific hardware frequency bands. It also means the wide elements of the signal can be used for different purposes. One algorithm can filter for ECG authentication while another filters differently for EMG etc.

In a preferred embodiment, when the determining of authentication information and biometric information takes place on the wearable computing means, the memory module 224 of the wearable computing means 402 may, for example, store a pre-enrolled user ECG signal for the determination of authentication and/or baseline user biometric information to compare with the measured biometric information.

The microprocessor 226 can process data received from the biometric sensors 105, 106 to authenticate and calculate accurate heart rate measurements, Heart Rate Variability measurements and user stress levels. The microprocessor 226 can also combine measurements from different biometric sensors to provide more accurate data to the user. Incorporating a number of algorithms, including ECG detection, BPM calculation, Heart Rate Variability measurements and calculating of user stress levels provides crucial information about the user's physiological state. Examples of suitable algorithms can be found in GB 1611963.8 the entirety of which is incorporated by reference.

The microprocessor 226 can cause the wearable apparatus 100 to automatically take readings from biometric sensors 105, 106 at regular intervals, on demand when requested from the remote terminal 200 or continually for a continuous authentication process. The remote terminal 200 can be any suitable electronic device. For example, the remote terminal may be a phone, tablet, desk top computer or laptop. Alternatively, the wearable apparatus 100 can be authenticated once and then kept in an authenticated state until removed.

The optional attachment means 102 can enable the wearable apparatus 100 to be temporarily secured where needed. The attachment means 102 can, for example, be clips, barb connections, buttons, press fasteners, hook and eye fasteners, fabric hook and loop fasteners or temporary adhesive.

The biometric sensors are incorporated into a garment and the wearable computing means are incorporated into the garment or carried with the user elsewhere. In a preferred embodiment, the wearable apparatus 100 can be a garment worn by the user, for example a compression top, compression leggings, sports bra, undergarments, shorts, shoes, socks or gloves or any garment having an elasticated strap or band worn by a user. In an alternative embodiment, the wearable apparatus 100 may be an accessory, for example a necklace, band, watch, belt, armband, bracelet or ring. In an alternative embodiment, the wearable apparatus 100 may be attached to a user garment, for example to elastic elements to enable good ECG signal acquisition by holding the biometric sensors 105, 106 against the skin. The wearable apparatus may be attached temporarily or permanently to any of the aforementioned by either being incorporated into the elastic or other elements of the garment/accessory design or by the optional attachment means 102. This can be beneficial for users that are not willing or able to wear a conventional device. The wearable apparatus 100 can be positioned on the chest area of the user, the small of the back and or the wrist areas for optimal signal acquisition.

### Other aspects, embodiments and modifications

The authentication information and the determined physiological state of the user may be securely stored on the memory module 224 of the wearable computing means 402. In this scenario, for example, the authentication information and the determined physiological state of the user can be transmitted when requested by the remote terminal 200 and/or stored for later analysis. In an example scenario, the stored biometric information may be useful in conjunction with a police body camera to analyse a police person's physiological state in response to certain events.

An authentication system comprising a plurality of biometric sensing devices can be incorporated in a seat and positioned to be close to a user's skin when seated in the seat, so as to capacitively measure the user's Electrocardiogram (ECG). The authentication system also has a means for communicating with a computing means and a computing means. The computing means is adapted to determine user authentication by comparing the user's ECG with a template. The seat is a seat of a vehicle and user authentication permits the user to start or control the vehicle. The sensing devices are located in a base of the seat and/or in the back of the seat. The authentication system also comprises filtering means for distinguishing the ECG signal from capacitive sensor changes resulting from the user shifting position in the seat.

## Claims

1. An authentication system (400) comprising:
a wearable apparatus (100), wherein the wearable apparatus (100) is a garment;
a wearable computing means (402) incorporated into the wearable apparatus (100), wherein the wearable computing means (402) comprises a microprocessor (226) and wireless communication means (240) for communicating with a remote terminal (200); and
a plurality of wearable biometric sensing devices (105, 106) incorporated into the wearable apparatus (100) and adapted to communicate with the wearable computing means (402),
wherein the plurality of wearable biometric sensing devices (105, 106) are electrodes adapted to measure a user's Electrocardiogram, ECG, and Electrodermal Activity, EDA,
wherein the plurality of wearable biometric sensing devices (105, 106) are ECG electrodes and EDA electrodes,
wherein the ECG electrodes and the EDA electrodes are the same electrodes,
wherein the plurality of wearable biometric sensing devices (105, 106) are incorporated in or on clothing in a manner in contact with skin,
wherein the wearable computing means (402) is adapted to determine user authentication by comparing the user's ECG with a template,
wherein the wearable computing means (402) is adapted to determine user physiological state by comparing the user's EDA with a threshold or profile based on measurements from one or more of the plurality of wearable biometric sensing devices (105, 106),
wherein the wearable computing means (402) is further adapted to utilize the determined physiological state and the user authentication to determine if the user should be authenticated when in a determined physiological state,
wherein the wearable computing means (402) is further adapted to activate a trigger when a user is authenticated but the determined user physiological state is outside a predetermined range, and
wherein the trigger activates at least one of an alarm, a camera, or initiating a communication to a remote service,
wherein the wearable computing means (402) further comprises a motion sensor (110) and wherein the trigger is suppressed when the determined user physiological state is outside a predetermined range and the motion sensor (110) detects a certain level of user motion.

2. The authentication system of claim 1, wherein the physiological state is at least one of user fatigue, duress or stress.

3. The authentication system of any preceding claim, wherein one or more of the plurality of wearable biometric sensing devices (105, 106) are common to both an amplifier for ECG measurement and an amplifier for EDA measurement.

4. The authentication system of any preceding claim, wherein the wearable computing means (402) is further adapted to determine when a user's EDA measurement is outside a predetermined range and to subsequently remove the user's authentication until the user authentication is re-determined.

5. The authentication system of any preceding claim, wherein the plurality of wearable biometric sensing devices (105, 106) are positioned close to the user's skin and measurements are taken capacitively.

6. The authentication system of claim 1, further comprising an authentication terminal as the remote terminal (200), the authentication terminal being adapted to perform an authentication process.

7. The authentication system of any preceding claim, wherein the biometric sensing devices (105, 106) are wirelessly connected to the wearable computing means (402).

8. A method of operating an authentication system (400) comprising:
measuring, by a plurality of wearable biometric sensing devices (105, 106) incorporated into a wearable apparatus (100), a user's Electrocardiogram, ECG, and Electrodermal Activity, EDA, wherein the plurality of wearable biometric sensing devices (105, 106) are electrodes, wherein the plurality of wearable biometric sensing devices (105, 106) are ECG electrodes and EDA electrodes, and wherein the ECG electrodes and the EDA electrodes are the same electrodes;
communicating, by the plurality of wearable biometric sensing devices (105, 106), with a wearable computing means (402) incorporated into the wearable apparatus (100), wherein the wearable computing means (402) comprises a microprocessor (226) and wireless communication means (240) for communicating with a remote terminal (200), wherein the wearable computing means (402) further comprises a motion sensor (110);
determining, by the wearable computing means (402), user authentication by comparing the user's ECG with a template;
determining, by the wearable computing means (402), user physiological state by comparing the user's EDA with a threshold or profile based on measurements from one or more of the plurality of wearable biometric sensing devices (105, 106);
utilizing the determined physiological state and the user authentication to determine if the user should be authenticated when in a determined physiological state; and
activating a trigger when a user is authenticated but the determined user physiological state is outside a predetermined range,
wherein the trigger activates at least one of an alarm, a camera, or initiating a communication to a remote service,
wherein the trigger is suppressed when the determined user physiological state is outside a predetermined range and the motion sensor (110) detects a certain level of user motion, and
wherein the plurality of wearable biometric sensing devices (105, 106) are incorporated in or on clothing in a manner in contact with skin and the wearable apparatus (100) is a garment.

## Patentansprüche

1. Ein Authentifizierungssystem (400), das Folgendes beinhaltet:
ein am Körper tragbares Gerät (100), wobei das am Körper tragbare Gerät (100) ein Kleidungsstück ist;
ein am Körper tragbares Rechenmittel (402), das in das am Körper tragbare Gerät (100) eingebaut ist, wobei das am Körper tragbare Rechenmittel (402) einen Mikroprozessor (226) und ein drahtloses Kommunikationsmittel (240) zum Kommunizieren mit einem abgesetzten Endgerät (200) beinhaltet; und
eine Vielzahl von am Körper tragbaren biometrischen Abtastvorrichtungen (105, 106), die in das am Körper tragbare Gerät (100) eingebaut sind und angepasst sind, um mit dem am Körper tragbaren Rechenmittel (402) zu kommunizieren,
wobei die Vielzahl von am Körper tragbaren biometrischen Abtastvorrichtungen (105, 106) Elektroden sind, die angepasst sind, um ein Elektrokardiogramm, EKG, und eine elektrodermale Aktivität, EDA, eines Benutzers zu messen,
wobei die Vielzahl von am Körper tragbaren biometrischen Abtastvorrichtungen (105, 106) EKG-Elektroden und EDA-Elektroden sind,
wobei die EKG-Elektroden und die EDA-Elektroden dieselben Elektroden sind,
wobei die Vielzahl von am Körper tragbaren biometrischen Abtastvorrichtungen (105, 106) in oder auf Kleidung in einer Haut berührenden Art und Weise eingebaut sind, wobei das am Körper tragbare Rechenmittel (402) angepasst ist, um eine Benutzerauthentifizierung durch Vergleichen des EKGs des Benutzers mit einem Muster zu bestimmen,
wobei das am Körper tragbare Rechenmittel (402) angepasst ist, um einen physiologischen Zustand des Benutzers durch Vergleichen der EDA des Benutzers mit einem Schwellenwert oder Profil basierend auf Messungen von einer oder mehreren der Vielzahl von am Körper tragbaren biometrischen Abtastvorrichtungen (105, 106) zu bestimmen,
wobei das am Körper tragbare Rechenmittel (402) ferner angepasst ist, um den bestimmten physiologischen Zustand und die Benutzerauthentifizierung zu nutzen, um zu bestimmen, ob der Benutzer authentifiziert werden sollte, wenn er sich in einem bestimmten physiologischen Zustand befindet,
wobei das am Körper tragbare Rechenmittel (402) ferner angepasst ist, um einen Auslöser zu aktivieren, wenn ein Benutzer authentifiziert ist, aber der bestimmte physiologische Zustand des Benutzers außerhalb eines vorbestimmten Bereichs liegt, und
wobei der Auslöser mindestens eines von einem Alarm, einer Kamera oder einem Einleiten einer Kommunikation mit einem abgesetzten Dienst aktiviert,
wobei das am Körper tragbare Rechenmittel (402) ferner einen Bewegungssensor (110) beinhaltet und wobei der Auslöser unterdrückt wird, wenn der bestimmte physiologische Zustand des Benutzers außerhalb eines vorbestimmten Bereichs liegt und der Bewegungssensor (110) einen gewissen Grad an Benutzerbewegung erkennt.

2. Authentifizierungssystem gemäß Anspruch 1, wobei der physiologische Zustand mindestens eines von Benutzererschöpfung, -belastung oder -anstrengung ist.

3. Authentifizierungssystem gemäß einem der vorhergehenden Ansprüche, wobei eine oder mehrere der Vielzahl von am Körper tragbaren biometrischen Abtastvorrichtungen (105, 106) sowohl einem Verstärker für EKG-Messungen als auch einem Verstärker für EDA-Messungen gemeinsam sind.

4. Authentifizierungssystem gemäß einem der vorhergehenden Ansprüche, wobei das am Körper tragbare Rechenmittel (402) ferner angepasst ist, um zu bestimmen, wenn eine EDA-Messung des Benutzers außerhalb eines vorbestimmten Bereichs liegt, und anschließend die Authentifizierung des Benutzers zu entfernen, bis die Authentifizierung des Benutzers neu bestimmt wird.

5. Authentifizierungssystem gemäß einem der vorhergehenden Ansprüche, wobei die Vielzahl von am Körper tragbaren biometrischen Abtastvorrichtungen (105, 106) nahe bei der Haut des Benutzers positioniert sind und Messungen kapazitiv abgelesen werden.

6. Authentifizierungssystem gemäß Anspruch 1, ferner beinhaltend ein Authentifizierungsendgerät als das abgesetzte Endgerät (200), wobei das Authentifizierungsendgerät angepasst ist, um einen Authentifizierungsprozess durchzuführen.

7. Authentifizierungssystem gemäß einem der vorhergehenden Ansprüche, wobei die biometrischen Abtastvorrichtungen (105, 106) mit dem am Körper tragbaren Rechenmittel (402) drahtlos verbunden sind.

8. Ein Verfahren zum Betätigen eines Authentifizierungssystems (400), das Folgendes beinhaltet:
Messen, durch eine Vielzahl von am Körper tragbaren biometrischen Abtastvorrichtungen (105, 106), die in ein am Körper tragbares Gerät (100) eingebaut sind, eines Elektrokardiogramms, EKG, und einer elektrodermalen Aktivität, EDA, eines Benutzers, wobei die Vielzahl von am Körper tragbaren biometrischen Abtastvorrichtungen (105, 106) Elektroden sind, wobei die Vielzahl von am Körper tragbaren biometrischen Abtastvorrichtungen (105, 106) EKG-Elektroden und EDA-Elektroden sind und wobei die EKG-Elektroden und die EDA-Elektroden dieselben Elektroden sind;
Kommunizieren, durch die Vielzahl von am Körper tragbaren biometrischen Abtastvorrichtungen (105, 106), mit einem am Körper tragbaren Rechenmittel (402), das in das am Körper tragbare Gerät (100) eingebaut ist, wobei das am Körper tragbare Rechenmittel (402) einen Mikroprozessor (226) und ein drahtloses Kommunikationsmittel (240) zum Kommunizieren mit einem abgesetzten Endgerät (200) beinhaltet, wobei das am Körper tragbare Rechenmittel (402) ferner einen Bewegungssensor (110) beinhaltet;
Bestimmen, durch das am Körper tragbare Rechenmittel (402), von einer Benutzerauthentifizierung durch Vergleichen des EKGs des Benutzers mit einem Muster;
Bestimmen, durch das am Körper tragbare Rechenmittel (402), eines physiologischen Zustands des Benutzers durch Vergleichen der EDA des Benutzers mit einem Schwellenwert oder Profil basierend auf Messungen von einer oder mehreren der Vielzahl von am Körper tragbaren biometrischen Abtastvorrichtungen (105, 106);
Nutzen des bestimmten physiologischen Zustands und der Benutzerauthentifizierung, um zu bestimmen, ob der Benutzer authentifiziert werden sollte, wenn er in einem bestimmten physiologischen Zustand ist; und
Aktivieren eines Auslösers, wenn ein Benutzer authentifiziert ist, aber der bestimmte physiologische Zustand des Benutzers außerhalb eines vorbestimmten Bereichs liegt, wobei der Auslöser mindestens eines von einem Alarm, einer Kamera oder einem Einleiten einer Kommunikation mit einem abgesetzten Dienst aktiviert,
wobei der Auslöser unterdrückt wird, wenn der bestimmte physiologische Zustand des Benutzers außerhalb eines vorbestimmten Bereichs liegt und der Bewegungssensor (110) einen gewissen Grad an Benutzerbewegung erkennt, und
wobei die Vielzahl von am Körper tragbaren biometrischen Abtastvorrichtungen (105, 106) in oder auf Kleidung in einer Haut berührenden Art und Weise eingebaut sind und das am Körper tragbare Gerät (100) ein Kleidungsstück ist.

## Revendications

1. Un système d'authentification (400) comprenant :
un appareil pouvant être porté sur soi (100), où l'appareil pouvant être porté sur soi (100) est un vêtement ;
un moyen informatique pouvant être porté sur soi (402) incorporé dans l'appareil pouvant être porté sur soi (100), où le moyen informatique pouvant être porté sur soi (402) comprend un microprocesseur (226) et un moyen de communication sans fil (240) pour communiquer avec un terminal à distance (200) ; et
une pluralité de dispositifs capteurs biométriques pouvant être portés sur soi (105, 106) incorporés dans l'appareil pouvant être porté sur soi (100) et conçus pour communiquer avec le moyen informatique pouvant être porté sur soi (402),
où la pluralité de dispositifs capteurs biométriques pouvant être portés sur soi (105, 106) sont des électrodes conçues pour mesurer l'électrocardiogramme, ECG, et l'activité électrodermale, AED, d'un utilisateur,
où la pluralité de dispositifs capteurs biométriques pouvant être portés sur soi (105, 106) sont des électrodes d'ECG et des électrodes d'AED,
où les électrodes d'ECG et les électrodes d'AED sont les mêmes électrodes,
où la pluralité de dispositifs capteurs biométriques pouvant être portés sur soi (105, 106) sont incorporés dans ou sur des éléments d'habillement d'une manière en contact avec la peau,
où le moyen informatique pouvant être porté sur soi (402) est conçu pour déterminer une authentification utilisateur par comparaison de l'ECG de l'utilisateur avec un modèle,
où le moyen informatique pouvant être porté sur soi (402) est conçu pour déterminer un état physiologique utilisateur par comparaison de l'AED de l'utilisateur avec un seuil ou un profil basé sur des mesures provenant d'un ou de plusieurs dispositifs de la pluralité de dispositifs capteurs biométriques pouvant être portés sur soi (105, 106),
où le moyen informatique pouvant être porté sur soi (402) est conçu en outre pour utiliser l'état physiologique déterminé et l'authentification utilisateur afin de déterminer si l'utilisateur devrait être authentifié quand il est dans un état physiologique déterminé,
où le moyen informatique pouvant être porté sur soi (402) est conçu en outre pour activer un déclencheur quand un utilisateur est authentifié mais que l'état physiologique utilisateur déterminé est hors d'une plage prédéterminée, et
où le déclencheur active au moins un élément parmi une alarme, une caméra, ou le lancement d'une communication avec un service à distance,
où le moyen informatique pouvant être porté sur soi (402) comprend en outre un capteur de mouvement (110) et où le déclencheur est supprimé quand l'état physiologique utilisateur déterminé est hors d'une plage prédéterminée et le capteur de mouvement (110) détecte un certain niveau de mouvement utilisateur.

2. Le système d'authentification de la revendication 1, où l'état physiologique est au moins un état parmi la fatigue, la contrainte ou le stress utilisateur.

3. Le système d'authentification de n'importe quelle revendication précédente, où un ou plusieurs dispositifs de la pluralité de dispositifs capteurs biométriques pouvant être portés sur soi (105, 106) sont communs à la fois à un amplificateur pour la mesure d'ECG et à un amplificateur pour la mesure d'AED.

4. Le système d'authentification de n'importe quelle revendication précédente, où le moyen informatique pouvant être porté sur soi (402) est conçu en outre pour déterminer quand la mesure d'AED d'un utilisateur est hors d'une plage prédéterminée et pour retirer par la suite l'authentification de l'utilisateur jusqu'à ce que l'authentification utilisateur soit redéterminée.

5. Le système d'authentification de n'importe quelle revendication précédente, où la pluralité de dispositifs capteurs biométriques pouvant être portés sur soi (105, 106) sont positionnés près de la peau de l'utilisateur et les mesures sont prises de manière capacitive.

6. Le système d'authentification de la revendication 1, comprenant en outre un terminal d'authentification comme terminal à distance (200), le terminal d'authentification étant conçu pour réaliser un processus d'authentification.

7. Le système d'authentification de n'importe quelle revendication précédente, où les dispositifs capteurs biométriques (105, 106) sont connectés sans fil au moyen informatique pouvant être porté sur soi (402).

8. Un procédé de mise en fonctionnement d'un système d'authentification (400) comprenant :
la mesure, par une pluralité de dispositifs capteurs biométriques pouvant être portés sur soi (105, 106) incorporés dans un appareil pouvant être porté sur soi (100), de l'électrocardiogramme, ECG, et de l'activité électrodermale, AED, d'un utilisateur, où la pluralité de dispositifs capteurs biométriques pouvant être portés sur soi (105, 106) sont des électrodes, où la pluralité de dispositifs capteurs biométriques pouvant être portés sur soi (105, 106) sont des électrodes d'ECG et des électrodes d'AED, et où les électrodes d'ECG et les électrodes d'AED sont les mêmes électrodes ;
la communication, par la pluralité de dispositifs capteurs biométriques pouvant être portés sur soi (105, 106), avec un moyen informatique pouvant être porté sur soi (402) incorporé dans l'appareil pouvant être porté sur soi (100), où le moyen informatique pouvant être porté sur soi (402) comprend un microprocesseur (226) et un moyen de communication sans fil (240) pour communiquer avec un terminal à distance (200), où le moyen informatique pouvant être porté sur soi (402) comprend en outre un capteur de mouvement (110) ;
la détermination, par le moyen informatique pouvant être porté sur soi (402), d'une authentification utilisateur par comparaison de l'ECG de l'utilisateur avec un modèle ;
la détermination, par le moyen informatique pouvant être porté sur soi (402), d'un état physiologique utilisateur par comparaison de l'AED de l'utilisateur avec un seuil ou un profil basé sur des mesures provenant d'un ou de plusieurs dispositifs de la pluralité de dispositifs capteurs biométriques pouvant être portés sur soi (105, 106) ;
l'utilisation de l'état physiologique déterminé et de l'authentification utilisateur afin de déterminer si l'utilisateur devrait être authentifié quand il est dans un état physiologique déterminé ; et
l'activation d'un déclencheur quand un utilisateur est authentifié mais que l'état physiologique utilisateur déterminé est hors d'une plage prédéterminée,
où le déclencheur active au moins un élément parmi une alarme, une caméra, ou le lancement d'une communication avec un service à distance,
où le déclencheur est supprimé quand l'état physiologique utilisateur déterminé est hors d'une plage prédéterminée et le capteur de mouvement (110) détecte un certain niveau de mouvement utilisateur, et
où la pluralité de dispositifs capteurs biométriques pouvant être portés sur soi (105, 106) sont incorporés dans ou sur des éléments d'habillement d'une manière en contact avec la peau et l'appareil pouvant être porté sur soi (100) est un vêtement.
